# EUROPEAN PATENT SPECIFICATION

(11) **EP 4 337 163 B1**
(45) Date of publication and mention of the grant of the patent: **18.02.2026**
(21) Application number: 22725642.7
(22) Date of filing: 06.05.2022
(51) Int. Cl.: A61K 8/73, A61Q 5/02, A61Q 5/12, C08B 37/02

(54) **HAIR CONDITIONER FORMULATION COMPRISING CATIONIC DEXTRAN POLYMER**
HAARKONDITIONIERUNGSFORMULIERUNG MIT KATIONISCHEM DEXTRANPOLYMER
FORMULATION DE CONDITIONNEUR CAPILLAIRE COMPRENANT UN POLYMÈRE DE DEXTRANE CATIONIQUE

(30) Priority: 12.05.2021 US 202163187599 P
(43) Date of publication of application: 20.03.2024
(73) Proprietor: Dow Silicones Corporation, Midland, MI 48686-0994 (US); Rohm and Haas Company, Collegeville, PA 19426 (US); Union Carbide Corporation, Seadrift, TX 77983 (US)
(72) Inventor: SUTHIWANGCHAROEN, Nisaraporn, Midland, Michigan 48686 (US); LEAL, Lyndsay M., Collegeville, Pennsylvania 19426 (US); BAI, Lu, Auburn, Michigan 48611 (US); PARTAIN III, Emmett M., Bound Brook, New Jersey 08805 (US); MILLER, Daniel S., Collegeville, Pennsylvania 19426 (US); REINER, Benjamin, Collegeville, Pennsylvania 19426 (US)
(74) Representative: Beck Greener LLP
(86) International application number: PCT/US2022/027985
(87) International publication number: WO 2022/240665

(56) References cited:
- CN-A- 108 056 942
- US-A1- 2009 197 784
- US-A1- 2010 093 584
- STANCIU MAGDALENA CRISTINA ET AL: "Influence of dextran hydrogel characteristics on adsorption capacity for anionic dyes", CARBOHYDRATE POLYMERS, APPLIED SCIENCE PUBLISHERS , LTD BARKING, GB, vol. 199, 5 July 2018 (2018-07-05), pages 75 - 83, XP085448310, ISSN: 0144-8617, DOI: 10.1016/J.CARBPOL.2018.07.011
- HAROLD FEIGENBAUM: "The Use of Cationizing Reagents in the Preparation of Conditioning Polymers for Hair and Skin Care", 3 March 2011 (2011-03-03), pages 1 - 10, XP055285791, Retrieved from the Internet <URL:https://web.archive.org/web/20110303225456/http://www.quab.com/files/Personal_Care_Article.pdf> [retrieved on 20160705]

## Description

The present invention relates to a hair conditioner formulation. In particular, the present invention relates to a hair conditioner formulation containing: a dermatologically acceptable vehicle; and a conditioning polymer, wherein the conditioning polymer is a cationic dextran polymer, comprising a dextran base polymer functionalized with quaternary ammonium groups; wherein the dextran base polymer has a weight average molecular weight of 10,000 to 3,000,000 Daltons; wherein the quaternary ammonium groups include (i) a quaternary ammonium group of formula (IIa) bound to a pendent oxygen on the dextran base polymer 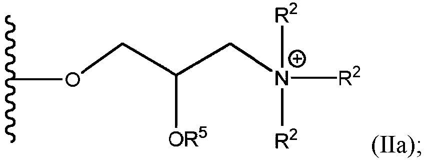 and
(ii) a quaternary ammonium group of formula (IIIa) bound to a pendent oxygen on the dextran base polymer 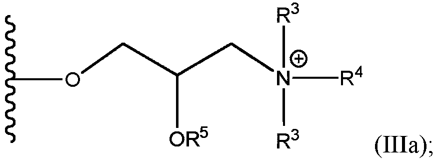
wherein 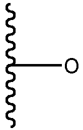 is a pendent oxygen on the dextran base polymer; wherein each R² is a methyl group; wherein each R³ is a methyl group;
and wherein each R⁴ is a linear or branched C₁₂ alkyl group; wherein each R⁵ is a hydrogen; wherein the conditioning polymer has a Kjeldahl nitrogen content corrected for ash and volatiles, TKN, of >1.0 to 5.0 wt%; and wherein the conditioning polymer has a cationic degree of substitution, DS, of dimethyldodecyl ammonium moieties of 0.01 to 0.03; wherein the hair conditioner formulation is selected from the group consisting of a leave on conditioner, a rinse off conditioner and a conditioning shampoo.

Conventional hair conditioners are popular with consumers for treating hair. Silicone based conditioning agents are the most commonly used conditioning agent in hair conditioner formulations. However, there are growing concerns among some consumers regarding the persistence and potential toxicity of certain silicone based conditioning agents or trace compounds incorporated with such silicone based conditioning agents in the environment, particularly for D4 and D5 conditioners. Accordingly, there has been a growing interest in the development of silicone-free alternative conditioning agents for use in hair conditioner formulations.

In U.S. Patent No. 5,879,670, Melby et al disclose a non-silicon containing amphyolyte polymer for use as a conditioning agent for treatment of a keratin-containing substrate. In particular, Melby et al disclose novel conditioning polymer containing (meth)acrylamidopropyltrimethyl ammonium chloride, meth(acrylic acid) or 2-(meth)acrylamido-2-methylpropane sulfonic acid and, optionally, a C₁₋₂₂ alkyl (meth)acrylate and the use thereof in a cosmetically acceptable medium for the treatment of a keratin-containing substrate (preferably, mammalian hair; more preferably, human hair).

US2010/0093584 discloses a non-cellulosic cationically modified polysaccharide in cleaning compositions.

"Influence of Dextran Hydrogel Characteristics on Adsorption Capacity for Anionic Dyes", Stanciu and Nichifor, Carbohydrate Polymers vol 199, pp75-83 discloses cationic amphiphilic dextran hydrogels with pendent N, N-dimethyl-N-alkyl-N-(2-hydroxypropyl) ammonium chloride groups.

Notwithstanding, there is a continuing need for new hair conditioning agents that provide conditioning benefits. There is also a continuing need for new hair conditioning agents having an increased natural origin index (ISO16128) when compared with conventional hair conditioning agents.

The present invention provides a hair conditioner formulation, comprising: a dermatologically acceptable vehicle; and a conditioning polymer, wherein the conditioning polymer is a cationic dextran polymer, comprising a dextran base polymer functionalized with quaternary ammonium groups; wherein the dextran base polymer has a weight average molecular weight of 10,000 to 3,000,000 Daltons; wherein the quaternary ammonium groups include (i) a quaternary ammonium group of formula (IIa) bound to a pendent oxygen on the dextran base polymer 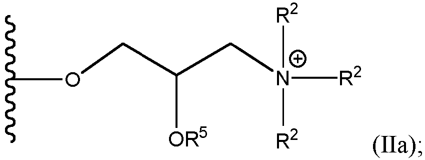 and
(ii) a quaternary ammonium group of formula (IIIa) bound to a pendent oxygen on the dextran base polymer 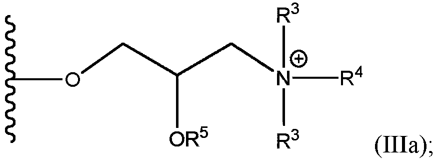
wherein 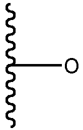 is a pendent oxygen on the dextran base polymer; wherein each R² is a methyl group; wherein each R³ is a methyl group; and wherein each R⁴ is a linear or branched C₁₂ alkyl group; wherein each R⁵ is a hydrogen; wherein the conditioning polymer has a Kjeldahl nitrogen content corrected for ash and volatiles, TKN, of >1.0 to 5.0 wt%; and wherein the conditioning polymer has a cationic degree of substitution, DS, of dimethyldodecyl ammonium moieties of 0.01 to 0.03; wherein the hair conditioner formulation is selected from the group consisting of a leave on conditioner, a rinse off conditioner and a conditioning shampoo.

The present invention provides a method of conditioning hair, comprising: selecting a hair conditioner formulation of the present invention; and applying the hair conditioner formulation to hair.

### DETAILED DESCRIPTION

We have surprisingly found that a cationic dextran polymer, comprising a dextran base polymer functionalized with quaternary ammonium groups; wherein the dextran base polymer has a weight average molecular weight of 10,000 to 3,000,000 Daltons; wherein the quaternary ammonium groups include (i) a quaternary ammonium group of formula (IIa) bound to a pendent oxygen on the dextran base polymer 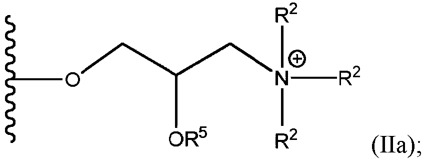 and
(ii) a quaternary ammonium group of formula (IIIa) bound to a pendent oxygen on the dextran base polymer 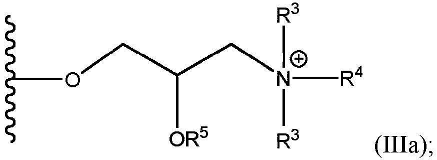
wherein 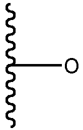 is a pendent oxygen on the dextran base polymer; wherein each R² is a methyl group; wherein each R³ is a methyl group; and wherein each R⁴ is a linear or branched C₁₂ alkyl group; wherein each R⁵ is a hydrogen; wherein the conditioning polymer has a Kjeldahl nitrogen content corrected for ash and volatiles, TKN, of >1.0 to 5.0 wt%; and wherein the conditioning polymer has a cationic degree of substitution, DS, of dimethyldodecyl ammonium moieties of 0.01 to 0.03 acts as a conditioning polymer that effectively restores hydrophobicity to damaged hair and reduces the force required to comb treated hair.

Unless otherwise indicated, ratios, percentages, parts, and the like are by weight.

As used herein, unless otherwise indicated, the phrase "molecular weight" or M_{W} refers to the weight average molecular weight as measured in a conventional manner with gel permeation chromatography (GPC) and conventional standards, such as polyethylene glycol standards. GPC techniques are discussed in detail in Modern Size Exclusion Chromatography, W. W. Yau, J. J. Kirkland, D. D. Bly; Wiley-Interscience, 1979, and in A Guide to Materials Characterization and Chemical Analysis, J. P. Sibilia; VCH, 1988, p.81-84. Molecular weights are reported herein in units of Daltons, or equivalently, g/mol.

The term "dermatologically acceptable" as used herein and in the appended claims refers to ingredients that are typically used for topical application to the skin, and is intended to underscore that materials that are toxic when present in the amounts typically found in skin care compositions are not contemplated as part of the present invention.

The term "damaged human hair" as used herein and in the appended claims refers to at least one of chemically damaged human hair (e.g., human hair damaged from chemical treatments such as dyeing, bleaching, perming); thermally damaged human hair (e.g., human hair damaged from exposure to heat via ironing, forced drying, styling); and physically damaged human hair (e.g., human hair damaged from physical abuse such as friction, pulling, curling).

Preferably, the hair conditioner formulation of the present invention is selected from the group consisting of a conditioning shampoo formulation, a rinse off conditioner formulation and a leave on conditioner formulation. More preferably, the hair conditioner formulation of the present invention is selected from the group consisting of a rinse off conditioner formulation and a leave on conditioner formulation. Most preferably, the hair conditioner formulation of the present invention is a rinse off conditioner formulation.

Preferably, the hair conditioner formulation of the present invention, comprises: a dermatologically acceptable vehicle (preferably, wherein the hair conditioner formulation comprises 25 to 99.9 wt% (more preferably, 48 to 99.85 wt%; still more preferably, 79 to 99.8 wt%; most preferably, 84.5 to 99.75 wt%), based on weight of the hair conditioner formulation, of the dermatologically acceptable vehicle); and a conditioning polymer (preferably, 0.05 to 5 wt% (more preferably, 0.1 to 2 wt%; still more preferably, 0.15 to 1 wt%; most preferably, 0.2 to 0.5 wt%), based on weight of the hair conditioner formulation, of the conditioning polymer), wherein the conditioning polymer is a cationic dextran polymer, comprising a dextran base polymer functionalized with quaternary ammonium groups; wherein the dextran base polymer has a weight average molecular weight of 10,000 to 3,000,000 Daltons; wherein the quaternary ammonium groups include (i) a quaternary ammonium group of formula (IIa) bound to a pendent oxygen on the dextran base polymer 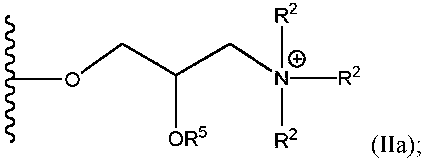 and
(ii) a quaternary ammonium group of formula (IIIa) bound to a pendent oxygen on the dextran base polymer 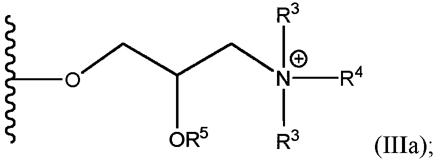
wherein 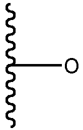 is a pendent oxygen on the dextran base polymer; wherein each R² is a methyl group; wherein each R³ is a methyl group; and wherein each R⁴ is a linear or branched C₁₂ alkyl group; wherein each R⁵ is a hydrogen; wherein the conditioning polymer has a Kjeldahl nitrogen content corrected for ash and volatiles, TKN, of >1.0 to 5.0 wt%; and wherein the conditioning polymer has a cationic degree of substitution, DS, of dimethyldodecyl ammonium moieties of 0.01 to 0.03.

Preferably, the hair conditioner formulation of the present invention is a liquid formulation. More preferably, the hair conditioner formulation of the present invention is an aqueous liquid formulation.

Preferably, the hair conditioner formulation of the present invention, comprises: 25 to 99.9 wt% (preferably, 48 to 99.85 wt%; more preferably, 79 to 99.8 wt%; most preferably, 84.5 to 99.75 wt%), based on weight of the hair conditioner formulation, of a dermatologically acceptable vehicle. More preferably, the hair conditioner formulation of the present invention, comprises: 25 to 99.9 wt% (preferably, 48 to 99.85 wt%; more preferably, 79 to 99.8 wt%; most preferably, 84.5 to 99.75 wt%), based on weight of the hair conditioner formulation, of a dermatologically acceptable vehicle; wherein the dermatologically acceptable vehicle comprises water. Still more preferably, the hair conditioner formulation of the present invention, comprises: 25 to 99.9 wt% (preferably, 48 to 99.85 wt%; more preferably, 79 to 99.8 wt%; most preferably, 84.5 to 99.75 wt%), based on weight of the hair conditioner formulation, of a dermatologically acceptable vehicle; wherein the dermatologically acceptable vehicle is selected from the group consisting of water and an aqueous C₁₋₄ alcohol mixture. Most preferably, the hair conditioner formulation of the present invention, comprises: 25 to 99.9 wt% (preferably, 48 to 99.85 wt%; more preferably, 79 to 99.8 wt%; most preferably, 84.5 to 99.75 wt%), based on weight of the hair conditioner formulation, of a dermatologically acceptable vehicle; wherein the dermatologically acceptable vehicle is water.

Preferably, the water used in the hair conditioner formulation of the present invention is at least one of distilled water and deionized water. More preferably, the water used in the hair conditioner formulation of the present invention is distilled and deionized.

Preferably, the hair conditioner formulation of the present invention, comprises: 0.05 to 5 wt% (preferably, 0.1 to 2 wt%; more preferably, 0.15 to 1 wt%; most preferably, 0.2 to 0.5 wt%), based on weight of the hair conditioner formulation, of a conditioning polymer; wherein the conditioning polymer is a cationic dextran polymer, comprising a dextran base polymer functionalized with quaternary ammonium groups; wherein the dextran base polymer has a weight average molecular weight of 10,000 to 3,000,000 Daltons; wherein the quaternary ammonium groups include (i) a quaternary ammonium group of formula (II) bound to a pendent oxygen on the dextran base polymer; and (ii) a quaternary ammonium group of formula (III) bound to a pendent oxygen on the dextran base polymer.

Preferably, the dextran base polymer has a weight average molecular weight of 10,000 to 3,000,000 Daltons (preferably, 50,000 to 2,000,000 Daltons; more preferably, 100,000 to 1,000,000 Daltons; still more preferably, 125,000 to 650,000 Daltons; most preferably, 145,000 to 525,000 Daltons). More preferably, the dextran base polymer has a weight average molecular weight of 10,000 to 3,000,000 Daltons (preferably, 50,000 to 2,000,000 Daltons; more preferably, 100,000 to 1,000,000 Daltons; still more preferably, 125,000 to 650,000 Daltons; most preferably, 145,000 to 525,000 Daltons); and the dextran base polymer is a branched chain dextran polymer comprising a plurality of glucose structural units; wherein 90 to 98 mol% (preferably, 92.5 to 97.5 mol%; more preferably, 93 to 97 mol%; most preferably, 94 to 96 mol%) of the glucose structural units are connected by α-D-1,6 linkages and 2 to 10 mol% (preferably, 2.5 to 7.5 mol%; more preferably, 3 to 7 mol%; most preferably, 4 to 6 mol%) of the glucose structural units are connected by α-1,3 linkages. Most preferably, the dextran base polymer has a weight average molecular weight of 10,000 to 3,000,000 Daltons (preferably, 50,000 to 2,000,000 Daltons; more preferably, 100,000 to 1,000,000 Daltons; still more preferably, 125,000 to 650,000 Daltons; most preferably, 145,000 to 525,000 Daltons); and the dextran base polymer is a branched chain dextran polymer comprising a plurality of glucose structural units; wherein 90 to 98 mol% (preferably, 92.5 to 97.5 mol%; more preferably, 93 to 97 mol%; most preferably, 94 to 96 mol%) of the glucose structural units are connected by α-D-1,6 linkages and 2 to 10 mol% (preferably, 2.5 to 7.5 mol%; more preferably, 3 to 7 mol%; most preferably, 4 to 6 mol%) of the glucose structural units are connected by α-1,3 linkages according to formula I 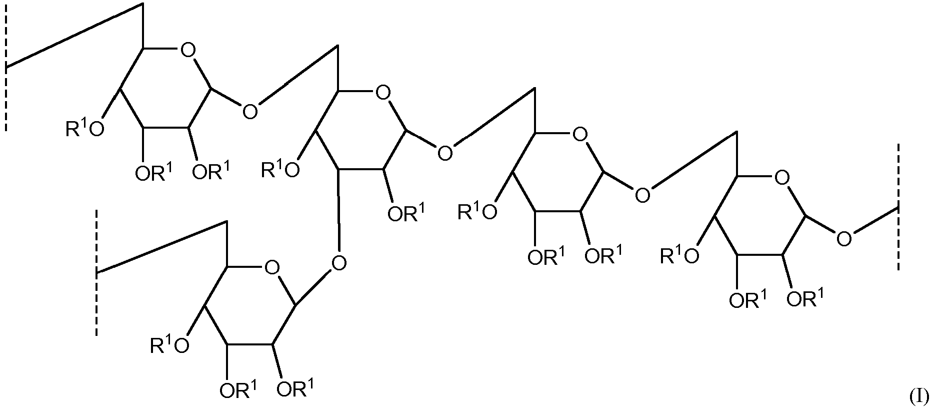 wherein R¹ is selected from a hydrogen, a C₁₋₄ alkyl group and a hydroxy C₁₋₄ alkyl group; and wherein the average branch off the dextran polymer backbone is ≤ 3 anhydroglucose units.

Preferably, the dextran base polymer contains less than 0.01 wt%, based on weight of the dextran base polymer, of alternan. More preferably, the dextran base polymer contains less than 0.001 wt%, based on weight of the dextran base polymer, of alternan. Most preferably, the dextran base polymer contains less than the detectable limit of alternan. More preferably, the hair conditioner formulation of the present invention comprises 0.05 to 5 wt% (preferably, 0.1 to 2 wt%; more preferably, 0.15 to 1 wt%; most preferably, 0.2 to 0.5 wt%), based on weight of the hair care formulation, of a conditioning polymer; wherein the conditioning polymer is a cationic dextran polymer, comprising a dextran base polymer functionalized with quaternary ammonium groups; wherein the quaternary ammonium groups include (i) a quaternary ammonium group of formula (IIa) bound to a pendent oxygen on the dextran base polymer 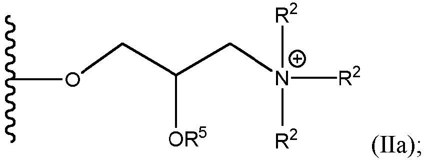 and
(ii) a quaternary ammonium group of formula (IIIa) bound to a pendent oxygen on the dextran base polymer 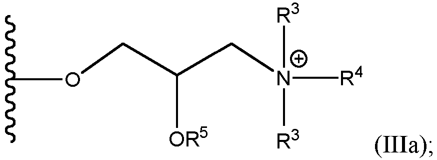
wherein 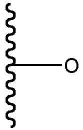 is a pendent oxygen on the dextran base polymer; wherein R⁵ is hydrogen; wherein each R² is a methyl group; wherein each R³ is a methyl group and wherein each R⁴ is a linear or branched C₁₂ alkyl group. Most preferably, the hair conditioner formulation of the present invention comprises 0.05 to 5 wt% (preferably, 0.1 to 2 wt%; more preferably, 0.15 to 1 wt%; most preferably, 0.2 to 0.5 wt%), based on weight of the hair conditioner formulation, of a conditioning polymer; wherein the conditioning polymer is a cationic dextran polymer, comprising a dextran base polymer functionalized with quaternary ammonium groups;
wherein the quaternary ammonium groups include (i) a quaternary ammonium group of formula (IIa) bound to a pendent oxygen on the dextran base polymer; and (ii) a quaternary ammonium group of formula (IIIa) bound to a pendent oxygen on the dextran base polymer; wherein each R² is a methyl group; wherein each R³ is a methyl group; wherein each R⁴ is independently selected from a linear or branched C₁₂ alkyl group; and wherein each R⁵ is a hydrogen.

The conditioning polymer has a Kjeldahl nitrogen content, TKN, of >1.0 to 5.0 wt% (preferably, >1.0 to 4 wt%; more preferably, >1.0 to 3 wt%; most preferably, 1.4 to 2.5 wt%) measured using a Buchi KjelMaster K-375 automated analyzer, corrected for volatiles and ash measured as described in ASTM method D-2364.

The conditioning polymer has a Kjeldahl nitrogen content, TKN, of > 1 to 5% (preferably, 1.4 to 2.5 wt% measured using a Buchi KjelMaster K-375 automated analyzer, corrected for volatiles and ash measured as described in ASTM method D-2364; wherein the dextran base polymer is functionalized with quaternary ammonium groups; wherein the quaternary ammonium groups include (i) a quaternary ammonium group of formula (IIa), and (ii) a quaternary ammonium group of formula (IIIa); wherein the conditioning polymer has a cationic degree of substitution, DS, of dimethyldodecyl ammonium moieties of 0.01 to 0.03 (preferably, 0.01 to < 0.03).

Preferably, the conditioning polymer comprises < 0.001 meq/gram (preferably, < 0.0001 meq/gram; more preferably, < 0.00001 meq/gram; most preferably, < detectable limit) of aldehyde functionality.

Preferably, the conditioning polymer comprises < 0.1 % (preferably, < 0.01 %; more preferably, < 0.001 %; most preferably, < detectable limit), of the linkages between individual glucose units in the conditioning polymer are β-1,4 linkages.

Preferably, the conditioning polymer comprises < 0.1 % (preferably, < 0.01 %; more preferably, < 0.001 %; most preferably, < detectable limit), of the linkages between individual glucose units in the conditioning polymer are β-1,3 linkages.

Preferably, the conditioning polymer comprises < 0.001 meq/gram (preferably, < 0.0001 meq/gram; more preferably, < 0.00001 meq/gram; most preferably, < detectable limit) of silicone containing functionality.

Preferably, the hair conditioner formulation of the present invention, optionally, further comprises at least one additional ingredient selected from the group consisting of a hair care cleaning surfactant; an antimicrobial agent/preservative (e.g., benzoic acid, sorbic acid, phenoxyethanol, methylisothiazolinone, ethylhexyl glycerin); a rheology modifier (e.g., PEG-150 pentaerythrityl tetrastearate); a colorant; pH adjusting agent; an antioxidant (e.g., butylated hydroxytoluene); a humectant (e.g., glycerin, sorbitol, monoglycerides, lecithins, glycolipids, fatty alcohols, fatty acids, polysaccharides, sorbitan esters, polysorbates (e.g., Polysorbate 20, Polysorbate 40, Polysorbate 60, and Polysorbate 80), diols (e.g., propylene glycol), diol analogs, triols, triol analogs, cationic polymeric polyols); a wax; a foaming agent; an emulsifying agent; a colorant; a fragrance; a chelating agent (e.g., tetrasodium ethylene diamine tetraacetic acid); a preservative (e.g., benzoic acid, sorbic acid, phenoxyethanol, methylisothiazolinone); a bleaching agent; a lubricating agent; a sensory modifier; a sunscreen additive; a vitamin; a protein/amino acid; a plant extract; a natural ingredient; a bioactive agent; an anti-aging agent; a pigment; an acid; a penetrant; an anti-static agent; an anti-frizz agent; an antidandruff agent; a hair waving/straightening agent; a hair styling agent; an absorbent; a hard particle; a soft particle; a conditioning agent (e.g., guar hydroxypropyltrimonium chloride, PQ-10, PQ-7); a slip agent; an opacifier; a pearlizing agent and a salt. More preferably, the hair conditioner formulation of the present invention, optionally, further comprises at least one additional ingredient selected from the group consisting of an antimicrobial agent/preservative (e.g., benzoic acid, sorbic acid, phenoxyethanol, methylisothiazolinone, ethylhexyl glycerin); a rheology modifier (e.g., PEG-150 pentaerythrityl tetrastearate); and a chelating agent (e.g., tetrasodium ethylene diamine tetraacetic acid). Most preferably, the hair conditioner formulation of the present invention, optionally, further comprises at least one additional ingredient selected from the group consisting of a mixture of phenoxyethanol and methylisothiazolinone; a mixture of phenoxyethanol and ethylhexyl glycerin; PEG-150 pentaerythrityl tetrastearate; and tetrasodium ethylene diamine tetraacetic acid.

Preferably, the hair conditioner formulation of the present invention is a conditioning shampoo formulation further comprising a hair cleaning surfactant. More preferably, the hair conditioner formulation of the present invention is a conditioning shampoo formulation, wherein the conditioning shampoo formulation comprises: 25 to 99.94 wt% (preferably, 48 to 98.9 wt%; more preferably, 79 to 97.35 wt%; most preferably, 84.5 to 94.8 wt%), based on weight of the conditioning shampoo formulation, of a vehicle; 0.05 to 5 wt% (preferably, 0.1 to 2 wt%; more preferably, 0.15 to 1 wt%; most preferably, 0.2 to 0.5 wt%), based on weight of the conditioning shampoo formulation, of a conditioning polymer as described herein above; and 0.01 to 74.95 wt% (preferably, 1 to 50 wt%; more preferably, 2.5 to 20 wt%; most preferably, 5 to 15 wt%), based on weight of the conditioning shampoo formulation, of a hair cleaning surfactant. Still more preferably, the hair conditioner formulation of the present invention is a conditioning shampoo formulation, wherein the conditioning shampoo formulation comprises: 25 to 99.94 wt% (preferably, 48 to 98.9 wt%; more preferably, 79 to 97.35 wt%; most preferably, 84.5 to 94.8 wt%), based on weight of the conditioning shampoo formulation, of a vehicle; 0.05 to 5 wt% (preferably, 0.1 to 2 wt%; more preferably, 0.15 to 1 wt%; most preferably, 0.2 to 0.5 wt%), based on weight of the conditioning shampoo formulation, of a conditioning polymer as described herein above; and 0.01 to 74.95 wt% (preferably, 1 to 50 wt%; more preferably, 2.5 to 20 wt%; most preferably, 5 to 15 wt%), based on weight of the conditioning shampoo formulation, of a hair cleaning surfactant; wherein the hair cleaning surfactant is selected from the group consisting of alkyl polyglucosides (e.g., lauryl glucoside, coco-glucoside, decyl glucoside), glycinates (e.g., sodium cocoyl glycinate), betaines (e.g., alkyl betaines such as cetyl betaine and amido betaines such as cocamidopropyl betaine), taurates (e.g., sodium methyl cocoyl taurate), glutamates (e.g., sodium cocoyl glutamate), sarcosinates (e.g., sodium lauroyl sarcosinate), isethionates (e.g., sodium cocoyl isethionate, sodium lauroyl methyl isethionate), sulfoacetates (e.g., sodium lauryl sulfoacetate), alaninates (e.g., sodium cocoyl alaninate), amphoacetates (e.g., sodium cocoamphoacetate), sulfates (e.g., sodium lauryl ether sulfate (SLES)), sulfonates (e.g., sodium C₁₄₋₁₆ olefin sulfonate), succinates (e.g., disodium lauryl sulfosuccinate), fatty alkanolamides (e.g., cocamide monoethanolamine, cocamide diethanolamine, soyamide diethanolamine, lauramide diethanolamine, oleamide monoisopropanolamine, stearamide monoethanolamine, myristamide monoethanolamine, lauramide monoethanolamine, capramide diethanolamine, ricinoleamide diethanolamine, myristamide diethanolamine, stearamide diethanolamine, oleylamide diethanolamine, tallowamide diethanolamine, lauramide monoisopropanolamine, tallowamide monoethanolamine, isostearamide diethanolamine, isostearamide monoethanolamine) and mixtures thereof. Yet more preferably, the hair conditioner formulation of the present invention is a conditioning shampoo formulation, wherein the conditioning shampoo formulation comprises: 25 to 99.94 wt% (preferably, 48 to 98.9 wt%; more preferably, 79 to 97.35 wt%; most preferably, 84.5 to 94.8 wt%), based on weight of the conditioning shampoo formulation, of a vehicle; 0.05 to 5 wt% (preferably, 0.1 to 2 wt%; more preferably, 0.15 to 1 wt%; most preferably, 0.2 to 0.5 wt%), based on weight of the conditioning shampoo formulation, of a conditioning polymer as described herein above; and 0.01 to 74.95 wt% (preferably, 1 to 50 wt%; more preferably, 2.5 to 20 wt%; most preferably, 5 to 15 wt%), based on weight of the conditioning shampoo formulation, of a hair cleaning surfactant; wherein the hair cleaning surfactant includes a sodium lauryl ether sulfate. Most preferably, the hair conditioner formulation of the present invention is a conditioning shampoo formulation, wherein the conditioning shampoo formulation comprises: 25 to 99.94 wt% (preferably, 48 to 98.9 wt%; more preferably, 79 to 97.35 wt%; most preferably, 84.5 to 94.8 wt%), based on weight of the conditioning shampoo formulation, of a vehicle; 0.05 to 5 wt% (preferably, 0.1 to 2 wt%; more preferably, 0.15 to 1 wt%; most preferably, 0.2 to 0.5 wt%), based on weight of the conditioning shampoo formulation, of a conditioning polymer as described herein above; and 0.01 to 74.95 wt% (preferably, 1 to 50 wt%; more preferably, 2.5 to 20 wt%; most preferably, 5 to 15 wt%), based on weight of the conditioning shampoo formulation, of a hair cleaning surfactant; wherein the hair cleaning surfactant includes a blend of a sodium lauryl ether sulfate, a cocamide monoethanolamine and a cocamidopropyl betaine.

Preferably, the hair conditioner formulation of the present invention is a conditioning shampoo formulation further comprising a hair cleaning surfactant; wherein the conditioning polymer has a Kjeldahl nitrogen content corrected for ash and volatiles, TKN, of 1.4 to 2.5 wt%; and wherein the conditioning polymer has a cationic degree of substitution, DS, of dimethyldodecyl ammonium moieties of 0.01 to 0.03.

Preferably, the hair conditioner formulation of the present invention further comprises a thickener. More preferably, the hair conditioner formulation of the present invention further comprises a thickener, wherein the thickener is selected to increase the viscosity of the hair conditioner formulation, preferably without substantially modifying the other properties of the hair conditioner formulation. Preferably, the hair conditioner formulation of the present invention further comprises a thickener, wherein the thickener is selected to increase the viscosity of the hair conditioner formulation, preferably without substantially modifying the other properties of the hair conditioner formulation and wherein the thickener accounts for 0 to 5.0 wt% (preferably, 0.1 to 5.0 wt %; more preferably, 0.2 to 2.5 wt %; most preferably, 0.5 to 2.0 wt%), based on weight of the hair conditioner formulation.

Preferably, the hair conditioner formulation of the present invention further comprises an antimicrobial agent/preservative. More preferably, the hair conditioner formulation of the present invention further comprises an antimicrobial/preservative, wherein the antimicrobial/preservative is selected from the group consisting of phenoxyethanol, ethylhexyl glycerin, benzoic acid, benzyl alcohol, sodium benzoate, DMDM hydantoin, 2-ethylhexyl glyceryl ether, isothiazolinone (e.g., methylchloroisothiazolinone, methylisothiazolinone) and mixtures thereof. Most preferably, the hair conditioner formulation of the present invention, further comprises an antimicrobial/preservative, wherein the antimicrobial/preservative is a mixture selected from the group consisting of (a) phenoxyethanol and ethylhexyl glycerin and (b) phenoxyethanol and an isothiazolinone (more preferably, wherein the antimicrobial/preservative is a mixture selected from the group consisting of (a) phenoxyethanol and ethylhexyl glycerin and (b) phenoxyethanol and methylisothiazolinone; most preferably, wherein the antimicrobial/preservative is a mixture of phenoxyethanol and ethylhexyl glycerin).

Preferably, the hair conditioner formulation of the present invention optionally further comprises a pH adjusting agent. More preferably, the hair conditioner formulation of the present invention, further comprises a pH adjusting agent, wherein the hair conditioner formulation has a pH of 4 to 9 (preferably, 4.25 to 8; more preferably, 4.5 to 7; most preferably, 4.75 to 6).

Preferably, the pH adjusting agent is selected from the group consisting of at least one of citric acid, lactic acid, hydrochloric acid, aminoethyl propanediol, triethanolamine, monoethanolamine, sodium hydroxide, potassium hydroxide, amino-2-methyl-1-propanol. More preferably, the pH adjusting agent is selected from the group consisting of at least one of citric acid, lactic acid, sodium hydroxide, potassium hydroxide, triethanolamine, amino-2-methyl-1-propanol. Still more preferably, the pH adjusting agent includes citric acid. Most preferably, the pH adjusting agent is citric acid.

Preferably, the hair conditioner formulation of the present invention, contains < 0.01 wt% (preferably, < 0.001 wt%; more preferably, < 0.0001 wt%; most preferably, < detectable limit), based on weight of the hair conditioner formulation of a dermatologically acceptable non-silicone oil. More preferably, the hair conditioner formulation of the present invention, contains < 0.01 wt% (preferably, < 0.001 wt%; more preferably, < 0.0001 wt%; most preferably, < detectable limit), based on weight of the hair conditioner formulation of a dermatologically acceptable non-silicone oil; wherein the dermatologically acceptable non-silicone oil is selected from the group consisting of hydrocarbon oils (e.g., mineral oil, petroleum jelly, polyisobutene, hydrogenated polyisobutene, hydrogenated polydecene, polyisohexadecane; natural oils (e.g., caprylic and capric triglyceride, sunflower oil, soybean oil, coconut oil, argan oil, olive oil, almond oil) and mixtures thereof.

Preferably, the hair conditioner formulation of the present invention contains < 0.1 wt% (preferably, < 0.001 wt%; more preferably, < 0.0001 wt%; most preferably, < detectable limit), based on weight of the hair conditioner formulation, of silicones (e.g., polydimethylsiloxanes, dimethicone, cyclodimethicone).

Preferably, the hair conditioner formulation of the present invention contains < 0.1 wt% (preferably, < 0.001 wt%; more preferably, < 0.0001 wt%; most preferably, < detectable limit), based on weight of the hair conditioner formulation, of silicon (Si) containing molecules.

Preferably, the hair conditioner formulation is selected from the group consisting of a leave on conditioner or rinse off conditioner; wherein the hair conditioner formulation contains < 0.1 wt% (preferably, < 0.001 wt%; more preferably, < 0.0001 wt%; most preferably, < detectable limit), based on weight of the hair conditioner formulation, of a hair care cleaning surfactant. More preferably, the hair conditioner formulation is selected from the group consisting of a leave on conditioner or rinse off conditioner; wherein the hair conditioner formulation contains < 0.1 wt% (preferably, < 0.001 wt%; more preferably, < 0.0001 wt%; most preferably, < detectable limit), based on weight of the hair conditioner formulation, of a hair care cleaning surfactant; wherein the hair cleaning surfactant is selected from the group consisting of alkyl polyglucosides (e.g., lauryl glucoside, coco-glucoside, decyl glucoside), glycinates (e.g., sodium cocoyl glycinate), betaines (e.g., alkyl betaines such as cetyl betaine and amido betaines such as cocamidopropyl betaine), taurates (e.g., sodium methyl cocoyl taurate), glutamates (e.g., sodium cocoyl glutamate), sarcosinates (e.g., sodium lauroyl sarcosinate), isethionates (e.g., sodium cocoyl isethionate, sodium lauroyl methyl isethionate), sulfoacetates (e.g., sodium lauryl sulfoacetate), alaninates (e.g., sodium cocoyl alaninate), amphoacetates (e.g., sodium cocoamphoacetate), sulfates (e.g., sodium lauryl ether sulfate (SLES)), sulfonates (e.g., sodium C₁₄₋₁₆ olefin sulfonate), succinates (e.g., disodium lauryl sulfosuccinate), fatty alkanolamides (e.g., cocamide monoethanolamine, cocamide diethanolamine, soyamide diethanolamine, lauramide diethanolamine, oleamide monoisopropanolamine, stearamide monoethanolamine, myristamide monoethanolamine, lauramide monoethanolamine, capramide diethanolamine, ricinoleamide diethanolamine, myristamide diethanolamine, stearamide diethanolamine, oleylamide diethanolamine, tallowamide diethanolamine, lauramide monoisopropanolamine, tallowamide monoethanolamine, isostearamide diethanolamine, isostearamide monoethanolamine) and mixtures thereof.

Preferably, the method of conditioning hair of the present invention comprises: selecting a hair conditioner formulation of the present invention and applying the hair conditioner formulation to the hair (preferably, mammalian hair; more preferably, human hair; most preferably, damaged human hair). More preferably, the method of conditioning hair of the present invention, comprises: selecting a hair conditioner formulation of the present invention; wetting the hair (preferably, mammalian hair; more preferably, human hair; most preferably, damaged human hair) with water; and applying the selected hair conditioner formulation to the wetted hair. Most preferably, the method of conditioning hair of the present invention, comprises: selecting a hair conditioner formulation of the present invention; wetting the hair (preferably, mammalian hair; more preferably, human hair; most preferably, damaged human hair) with water; applying the selected hair conditioner formulation to the wetted hair; and then rinsing the hair with water.

Some embodiments of the present invention will now be described in detail in the following **Examples.**

### Synthesis S1: Synthesis of Cationic Dextran Polymer

A 500 mL, four necked, round bottom flask fitted with a rubber serum cap, a nitrogen inlet, a pressure equalizing addition funnel, a stirring paddle and motor, a subsurface thermocouple connected to a J-KEM controller and a Friedrich condenser connected to a mineral oil bubbler was charged with dextran base polymer (30.29 g; from BOC Scientific) and deionized water (140.19 g). The addition funnel was charged with a 70% aqueous solution of 2,3-epoxypropyltrimethylammonium chloride (27.19 g; QUAB^{®} 151 available from SKW QUAB Chemicals). The flask contents were allowed to stir until the dextran base polymer dissolved in the deionized water. While the contents were stirring, the apparatus was purged with nitrogen to displace any oxygen entrained in the system. The nitrogen flow rate was about 1 bubble per second. The mixture was purged with nitrogen while stirring for one hour. Using a plastic syringe, a 25% aqueous sodium hydroxide solution (4.77 g) was added over a period of a few minutes to the flask contents with stirring under nitrogen. The flask contents were then allowed to stir under nitrogen for 30 minutes. The contents of the addition funnel were then charged to the flask contents dropwise over a few minutes under nitrogen with continued stirring. After the contents of the addition funnel were transferred to the flask contents, the mixture was allowed to stir for 5 minutes. Then heat was applied to the flask contents with a heating mantle controlled using the J-KEM controller set at 55 °C. The flask contents were heated to and maintained at 55 °C for 90 minutes. The flask contents were then cooled to room temperature while maintaining a positive nitrogen pressure in the flask. When the flask contents reached room temperature, glacial acetic acid (2.0 g) was added. The polymer was recovered by non-solvent precipitation from methanol, recovering the precipitated polymer by vacuum filtration using a Buchner funnel and dried overnight *in vacuo* at 50 °C. The product branched chain cationic dextran polymer was an off-white solid (34.3 g), with a volatiles content of 2.72%, an ash content of 0.37% (as sodium chloride). The volatiles and ash were measured as described in ASTM method D-2364. The Kjeldahl nitrogen content, TKN, was measured using a Buchi KjelMaster K-375 automated analyzer, and was found to be 1.49% (corrected for volatiles and ash), which corresponds to a trimethylammonium degree of substitution of 0.21.

### Synthesis S2: Synthesis of Cationic Dextran Polymer

A 500 mL, four necked, round bottom flask fitted with a rubber serum cap, a nitrogen inlet, a pressure equalizing addition funnel, a stirring paddle and motor, a subsurface thermocouple connected to a J-KEM controller and a Friedrich condenser connected to a mineral oil bubbler was charged with dextran base polymer (25.0 g; Sigma Aldrich catalog # D4876) and deionized water (100 g). The contents of the flask were stirred at 70 rpm. While stirring, the head space in the flask was purged with a slow, steady flow of nitrogen (about one bubble per second) for one hour to remove any entrained oxygen in the apparatus.

The addition funnel was charged with a 70% aqueous solution of 2,3-epoxypropyltrimethylammonium chloride (30.1 g; QUAB^{®} 151 available from SKW QUAB Chemicals) and a 40% aqueous solution of 3-chloro-2-hydroxypropyl-lauryl-dimethylammonium chloride (39.4 g; QUAB^{®} 342 available from SKW QUAB Chemicals).

While stirring the flask contents under nitrogen, a 25% aqueous sodium hydroxide solution (11.4 g) was added to the flask contents over 2 minutes. The flask contents were then continually stirred for one hour before adding the contents of the addition funnel to the flask contents dropwise over 3 minutes. The flask contents were then stirred for 20 minutes before heating the flask contents using a heating mantle with a set point temperature of 55 °C for 1.5 hours. The flask contents were then cooled in an ice water bath while maintaining a positive nitrogen pressure in the flask. The flask contents were then neutralized by adding glacial acetic acid (1.66 g) to the flask contents. The flask contents were then stirred for 10 minutes under nitrogen. A polymer product was then recovered from the flask contents by non-solvent precipitation in methanol; roughly 1 L of methanol was used. The methanol was then decanted off and the polymer product was placed in a dish and dried *in vacuo* at 50 °C overnight.

The polymer product recovered was sieved through a 30 mesh screen and obtained as a free-flowing white solid (27.2 g) with a volatiles content of 5.13% and an ash content (as sodium acetate) of 0.56%. The total Kjeldahl nitrogen in the polymer product was determined to be 1.329 wt%.

### Synthesis S3: Synthesis of Cationic Dextran Polymer

In **Synthesis S3,** cationic dextran polymer was prepared substantially as described in **Synthesis S2** but with varying reagent feeds as noted in **TABLE 1.** The degree of cationic substitution, CS, of the QUAB^{®} 151 and of the QUAB^{®} 342 in the product cationic dextran polymers measured by NMR is reported in **TABLE 2.** The total Kjeldahl nitrogen, TKN, in the product cationic dextran polymers is also reported in **TABLE 2.**

**TABLE 1**

| **Example** | **Dextran (g)** | **NaOH (g)** | **QUAB^{®} 151 (g)** | **QUAB^{®} 342 (g)** |
|---|---|---|---|---|
| **Synthesis S2** | 25.0 | 11.4 | 30.1 | 39.4 |
| **Synthesis S3** | 25.1 | 4.6 | 30.0 | 4.9 |

**TABLE 2**

| **Ex.** | **Degree of cationic substitution, CS** | | | **TKN (wt%)** |
|---|---|---|---|---|
| | **QUAB^{®} 151** | **QUAB^{®} 342** | **Total** | |
| **Synthesis S2** | 0.121 | 0.013 | 0.134 | 1.329 |
| **Synthesis S3** | 0.141 | 0.005 | 0.146 | 1.706 |

### Comparative Examples CF1-CF3 and Example F1: Hair Conditioner Formulations

A hair conditioner formulation was prepared in each of **Comparative Examples CF1-CF3** and **Example F1** having the formulation noted in **TABLE 3.**

**TABLE 3**

| **Ingredient INCI name** | **CF1** | **CF2** | **CF3** | **F1** |
|---|---|---|---|---|
| | **wt%** | **wt%** | **wt%** | **wt%** |
| Deionized water | q.s. 100 | | | |
| Hydroxyethyl cellulose¹ | 1.4 | 1.4 | 1.4 | 1.4 |
| Tetrasodium EDTA² | 0.2 | 0.2 | 0.2 | 0.2 |
| Cetearyl alcohol³ | 1.0 | 1.0 | 1.0 | 1.0 |
| Glyceryl stearate (and) PEG-100 stearate⁴ | 1.0 | 1.0 | 1.0 | 1.0 |
| Unmodified branched chain dextran⁵ | 0 | 0.3 | 0 | 0 |
| Polymer product of **Synthesis S1** | 0 | 0 | 0.3 | 0 |
| Polymer product of **Synthesis S2** | 0 | 0 | 0 | 0.3 |
| Phenoxyethanol and Methylisothiazolinone⁶ | 0.5 | 0.5 | 0.5 | 0.5 |
| ¹ available from The Dow Chemical Company under tradename Cellosize^{™} PCG-10 | | | | |
| ² available from The Dow Chemical Company under tradename Versene^{™} 220 | | | | |
| ³ available from Croda Inc. under tradename Crodacol^{™} CS50 | | | | |
| ⁴ available from Croda Inc. under tradename Arlacel^{™} 165 | | | | |
| ⁵ available from Sigma Aldrich under catalog number D4876 | | | | |
| ⁶ preservative available from The Dow Chemical Company under tradename Neolone^{™} PE | | | | |

### Hair conditioning performance

Studies to evaluate ease of wet and dry combing of hair treated with a rinse off conditioner formulation of **Comparative Examples CF1-CF3** and **Example F1** were performed as follows. Slightly bleached Caucasian hair from International Hair Importers was used for testing the conditioners. Each tress weighed 2 grams. Each tress was rinsed for 30 seconds under a stream of 40 °C tap water. Using a pipette, 0.4 grams of a solution containing nine percent of sodium lauryl sulfate was applied and lathered through each tress for 30 seconds. The tresses were then rinsed for 1 minute under running water. Excess water was removed from the tresses by passing each tress between the index and middle fingers of the hand. The tresses were then treated with a rinse off conditioner formulation of **Comparative Examples CF1-CF3** and **Example F1** at 0.4 g formulation/g of hair by massaging the formulation into the wet/damp hair for 1 minute. The tresses were rinsed for 30 seconds under running water and dried overnight at room temperature.

An INSTRON Model 4464 running BlueHill 2 software was also used for determining conditioning performance by the ease of wet combing and the ease of dry combing. The test employed an INSTRON strain gauge, which was equipped to measure the force required to comb the hair. The conditioning performance was based on the ability of the rinse off conditioner formulation, to reduce the force required to comb the hair with the INSTRON strain gauge. The force was reported as an Average Combing Load (ACL). The lower the number of the ACL value, the better the conditioning effect imparted by the rinse off conditioner formulation tested.

According to the INSTRON wet combing method, hair was first wetted by dipping into distilled water, and then the hair was detangled by combining the tress three times. The tress was then retangled by dipping in distilled water three times. Excess water was removed by passing the tress through the index and middle fingers of the hand twice. The tress was placed on a hanger and INSTRON combed. An average wet combing force from three tresses was measured for each rinse off conditioner formulation. The average wet combing results are provided in **TABLE 4.**

According to the INSTRON dry combining method, dry hair was detangled by combining the tress 3 times. Then the hair was retangled by swirling the tress clockwise 3 times and swirling it counter clockwise 3 times. The tress was then placed on a hanger and INSTRON combed. An average dry combining force from three tresses was measured for each rinse off conditioner formulation. The average dry combining results are provided in **TABLE 4.**

**TABLE 4**

| **Rinse off Conditioner** | **ACL (kgf)** | | |
|---|---|---|---|
| | **Dry** | **Wet** | **Dry + Wet** |
| **Comparative Example CF1** | 0.0462 | 0.8684 | 0.9146 |
| **Comparative Example CF2** | 0.0636 | 0.7623 | 0.8259 |
| **Comparative Example CF3** | 0.0245 | 0.4880 | 0.5125 |
| **Example F1** | 0.0275 | 0.2984 | 0.3259 |

### Hair Hydrophobicity

Rinse off hair conditioner prepared according to each of **Comparative Examples CF2-CF3** and **Example F1** were tested on two separate 3 g hair samples (Slightly Bleached Caucasian Hair, International Hair Importers, Inc.). The hair samples were first rinsed with water for 30 seconds. Then a 9% w/w aqueous solution of sodium lauryl sulfate was massaged into the hair samples for 30 seconds. Then the hair samples were rinsed with water for 60 seconds. The hair samples were then treated with the rinse off hair conditioner at a dosage of 0.4 g/g or hair and massaged onto the hair for 30 seconds. The hair samples where then rinsed with water for 30 seconds and dried before hydrophobicity testing.

To measure hydrophobicity of the hair, the tresses were combed straight and then held tightly on both ends with a holder. Ten 30 µL drops of water were placed at different locations on each tress from the root to the tip. While the water was observed to immediately dissipate into the tresses treated with the formulation of **Comparative Example CF2 or CF3,** the water continued to bead off of the tress treated with the formulation of **Example F1** even after 3 minutes indicating that the rinse off conditioner formulation of **Example F1** successfully restored hydrophobicity benefit to the slightly bleached Caucasian hair.

### Comparative Examples CF4-CF6 and Example F2: Shampoo Conditioner Formulations

A shampoo conditioner formulation was prepared in each of **Comparative Examples CF4-CF6** and **Example F2** having the formulation noted in **TABLE 5.**

**TABLE 5**

| **Ingredient INCI name** | **CF4** | **CF5** | **CF6** | **F2** |
|---|---|---|---|---|
| | **wt%** | **wt%** | **wt%** | **wt%** |
| Deionized water | q.s. 100 | | | |
| Sodium lauryl ether sulfate¹ | 9 | 9 | 9 | 9 |
| Guar hydroxypropyltrimonium chloride² | 0 | 0.3 | 0 | 0 |
| Conditioning polymer from **Synthesis S2** | 0 | 0 | 0 | 0.3 |
| Conditioning polymer from **Synthesis S3** | 0 | 0 | 0.3 | 0 |
| Tetrasodium EDTA³ | 0.2 | 0.2 | 0.2 | 0.2 |
| Cocamide MEA⁴ | 1.0 | 1.0 | 1.0 | 1.0 |
| Cocamidopropyl Betaines | 2.1 | 2.1 | 2.1 | 2.1 |
| Phenoxyethanol and Methylisothiazolinone⁶ | 0.25 | 0.25 | 0.25 | 0.25 |
| PEG-150 Pentaerythrityl Tetrastearate⁷ | q.s. viscosity 11,000 cP | | | |
| Sodium hydroxide or Citric acid | q.s. pH 5 | | | |
| ¹ available from Stepan Company under tradename STEOL^{®} CS-130 | | | | |
| ² available from Solvay Novecare under tradename JAGUAR^{®} Excel | | | | |
| ³ available from The Dow Chemical Company under tradename VERSENE^{™} 220 | | | | |
| ⁴ available from Croda Inc. under tradename INCROMIDE^{™} CMEA | | | | |
| ⁵ available from Stepan Company under tradename AMPHOSOL^{®} CA | | | | |
| ⁶ preservative available from The Dow Chemical Company under tradename Neolone^{™} PE | | | | |
| ⁷ available from Croda Inc. under tradename Crothix^{™}-PA-(MH) | | | | |

### Hair conditioning performance

Studies to evaluate ease of wet and dry combing of hair treated with conditioning shampoo formulation of **Comparative Examples CF4-CF6** and **Example F2** were performed as follows. Slightly bleached Caucasian ("SBC") hair and Virgin brown ("VB") hair from International Hair Importers were used for the testing. Each tress weighed 2 grams. Each tress was rinsed for 15 seconds under a stream of 40 °C tap water. Using a pipette, 0.2 grams of a solution containing nine percent of sodium lauryl sulfate was applied and lathered through each tress for 30 seconds. The tresses were then rinsed for 1 minute under running water. Excess water was removed from the tresses by passing each tress between the index and middle fingers of the hand. The tresses were then treated with a conditioning shampoo formulation of **Comparative Examples CF4-CF6** and **Example F2** at 0.1 g formulation/g of hair by massaging the formulation into the wet/damp hair for 1 minute. The tresses were placed on a tray covered with paper towels and dried overnight at room temperature.

Wet combing results for hair tresses treated with the conditioning shampoo formulations of **Comparative Examples CF4-CF6** and **Example F2** were determined as follows. The hair tress was dipped one time in deionized water. Excess water was removed from the tress by pulling through the index and middle fingers twice. Each tress was then a combed until tangles were removed. Each tress was then retangled by dipping in distilled water three times at roughly once per second frequency. Excess water was removed by passing through the index and middle fingers twice. Then, with a comb in place on a Diastron MTT175 tensile tester with 50 g normal force mounted on rubber probe was used for testing in a temperature and humidity controlled room. Three tresses per treatment and four measurements per tress were tested to generate the total mean work in joules reported in **TABLE 6.**

**TABLE 6**

| **Conditioning Shampoo Formulation** | **Hair** | **Total work (J)** |
|---|---|---|
| **Comparative Example CF4** | SBC | 0.85 |
| **Comparative Example CF4** | VB | 0.33 |
| **Comparative Example CF5** | SBC | 0.36 |
| **Comparative Example CF5** | VB | 0.19 |
| **Comparative Example CF6** | SBC | 1.00 |
| **Comparative Example CF6** | VB | 0.47 |
| **Example F2** | SBC | 0.39 |
| **Example F2** | VB | 0.25 |

## Claims

1. A hair conditioner formulation, comprising:
a dermatologically acceptable vehicle; and
a conditioning polymer, wherein the conditioning polymer is a cationic dextran polymer, comprising a dextran base polymer functionalized with quaternary ammonium groups; wherein the dextran base polymer has a weight average molecular weight of 10,000 to 3,000,000 Daltons; wherein the quaternary ammonium groups include
(i) a quaternary ammonium group of formula (IIa) bound to a pendent oxygen on the dextran base polymer 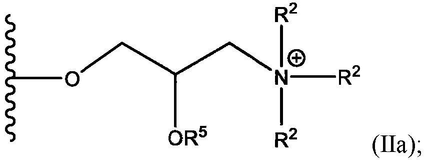 and
(ii) a quaternary ammonium group of formula (IIIa) bound to a pendent oxygen on the dextran base polymer 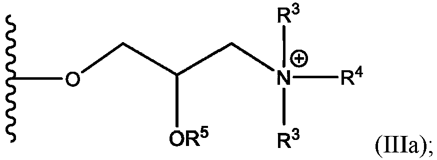
wherein 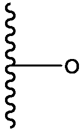 is a pendent oxygen on the dextran base polymer; wherein each R² is a methyl group; wherein each R³ is a methyl group;
wherein each R⁴ is a linear or branched C₁₂ alkyl group; wherein each R⁵ is a hydrogen;
wherein the conditioning polymer has a Kjeldahl nitrogen content corrected for ash and volatiles, TKN, of >1.0 to 5.0 wt%; and wherein the conditioning polymer has a cationic degree of substitution, DS, of dimethyldodecyl ammonium moieties of 0.01 to 0.03; wherein the hair conditioner formulation is selected from the group consisting of a leave on conditioner, a rinse off conditioner and a conditioning shampoo.

2. The hair conditioner formulation of claim 1, wherein the hair conditioner formulation contains less than 0.01 wt%, based on weight of the hair conditioner formulation, of a dermatologically acceptable oil.

3. The hair conditioner formulation of claim 1, wherein the hair conditioner formulation contains less than 0.1 wt%, based on weight of the hair conditioner formulation, of silicon containing molecules.

4. The hair conditioner formulation of claim 1, further comprising at least one additional ingredient selected from the group consisting of an antimicrobial agent/preservative; a hair care cleaning surfactant; a rheology modifier; a soap; a colorant; pH adjusting agent; an antioxidant; a humectant; a wax; a foaming agent; an emulsifying agent; a colorant; a fragrance; a chelating agent; a preservative; a bleaching agent; a lubricating agent; a sensory modifier; a sunscreen additive; a vitamin; a protein/amino acid; a plant extract; a natural ingredient; a bioactive agent; an anti-aging agent; a pigment; an acid; a penetrant; an anti-static agent; an anti-frizz agent; an antidandruff agent; a hair waving/straightening agent; a hair styling agent; an absorbent; a hard particle; a soft particle; a conditioning agent; a slip agent; an opacifier; a pearlizing agent and a salt.

5. The hair conditioner formulation of claim 1, further comprising a hair care cleaning surfactant; wherein the conditioning polymer has a Kjeldahl nitrogen content corrected for ash and volatiles, TKN, of 1.4 to 2.5 wt%; and wherein the conditioning polymer has a cationic degree of substitution, DS, of dimethyldodecyl ammonium moieties of 0.01 to 0.03.

6. A method of conditioning hair, comprising:
selecting a hair conditioner formulation according to claim 1; and
applying the hair conditioner formulation to hair.

## Patentansprüche

1. Haarkonditionierungsformulierung, umfassend:
eine dermatologisch verträgliche Trägersubstanz; und
ein Konditionierungspolymer, wobei das Konditionierungspolymer ein kationisches Dextranpolymer ist, umfassend ein mit quartären Ammoniumgruppen funktionalisiertes Dextranbasispolymer; wobei das Dextranbasispolymer ein gewichtsmittleres Molekulargewicht von 10.000 bis 3.000.000 Dalton aufweist; wobei die quartären Ammoniumgruppen einschließen
(i) eine quartäre Ammoniumgruppe der Formel (IIa), die an einen anhängenden Sauerstoff an dem Dextranbasispolymer gebunden ist 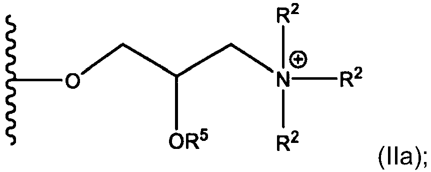 und
(ii) eine quartäre Ammoniumgruppe der Formel (IIIa), die an einen anhängenden Sauerstoff an dem Dextranbasispolymer gebunden ist 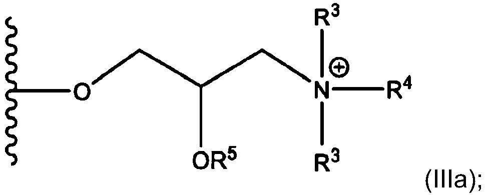
wobei 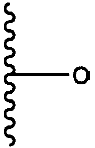 ein anhängender Sauerstoff an dem Dextranbasispolymer ist;
wobei jedes R² eine Methylgruppe ist; wobei jedes R³ eine Methylgruppe ist;
wobei jedes R⁴ eine lineare oder verzweigte C₁₂-Alkylgruppe ist; wobei jedes R⁵ ein Wasserstoff ist; wobei das Konditionierungspolymer einen um Asche und flüchtige Stoffe korrigierten Kjeldahl-Stickstoffgehalt, TKN, von >1,0 bis 5,0 Gew.-% aufweist; und wobei das Konditionierungspolymer einen kationischen Substitutionsgrad, DS, von Dimethyldodecylammoniumeinheiten von 0,01 bis 0,03 aufweist; wobei die Haarkonditionierungsformulierung aus der Gruppe ausgewählt ist, die aus einem Leave-on-Konditionierungsmittel, einem Spül-Konditionierungsmittel und einem konditionierenden Shampoo besteht.

2. Haarkonditionierungsformulierung nach Anspruch 1, wobei die Haarkonditionierungsformulierung zu weniger als 0,01 Gew.-%, bezogen auf das Gewicht der Haarkonditionierungsformulierung, ein dermatologisch verträgliches Öl enthält.

3. Haarkonditionierungsformulierung nach Anspruch 1, wobei die Haarkonditionierungsformulierung zu weniger als 0,1 Gew.-%, bezogen auf das Gewicht der Haarkonditionierungsformulierung, siliciumhaltige Moleküle enthält.

4. Haarkonditionierungsformulierung nach Anspruch 1, ferner umfassend mindestens einen zusätzlichen Inhaltsstoff, der ausgewählt ist aus der Gruppe bestehend aus einem antimikrobiellen Mittel/Konservierungsmittel; einem Haarpflegereinigungstensid; einem Rheologiemodifizierer; einer Seife; einem Farbstoff; einem pH-Wert-Einstellmittel; einem Antioxidationsmittel; einem Feuchthaltemittel; einem Wachs; einem Schaummittel; einem Emulgiermittel; einem Farbstoff; einem Duft; einem Chelatbildner; einem Konservierungsmittel; einem Bleichmittel; einem Schmiermittel; einem sensorischen Modifikator; einem Sonnenschutzzusatz; einem Vitamin; einem Protein/einer Aminosäure; einem Pflanzenextrakt; einem natürlichen Bestandteil; einem bioaktiven Wirkstoff; einem Alterungsschutzmittel; einem Pigment; einer Säure; einem Eindringmittel; einem Antistatikum; einem Anti-Frizz-Mittel; einem Antischuppenmittel; einem Mittel zum Wellen/Glätten von Haaren; einem Haarstylingmittel; einem Absorptionsmittel; einem hartem Partikel; einem weichen Partikel; einem Konditionierungsmittel; einem Gleitmittel; einem Trübungsmittel; einem Perlglanzmittel und einem Salz.

5. Haarkonditionierungsformulierung nach Anspruch 1, ferner umfassend ein Haarpflegereinigungstensid; wobei das Konditionierungspolymer einen um Asche und flüchtige Stoffe korrigierten Kjeldahl-Stickstoffgehalt, TKN, von 1,4 bis 2,5 Gew.-% aufweist; und wobei das Konditionierungspolymer einen kationischen Substitutionsgrad, DS, von Dimethyldodecylammoniumeinheiten von 0,01 bis 0,03 aufweist.

6. Verfahren zum Konditionieren von Haar, umfassend:
Auswählen einer Haarkonditionierungsformulierung nach Anspruch 1; und Auftragen der Haarkonditionierungsformulierung auf Haar.

## Revendications

1. Formulation de conditionneur de cheveux, comprenant :
un véhicule dermatologiquement acceptable ; et
un polymère de conditionnement, dans laquelle le polymère de conditionnement est un polymère de dextrane cationique, comprenant un polymère de base de dextrane fonctionnalisé avec des groupes ammonium quaternaire ; dans laquelle le polymère de base de dextrane a une masse moléculaire moyenne en poids de 10 000 à 3 000 000 Daltons ; dans laquelle les groupes ammonium quaternaire comportent
(i) un groupe ammonium quaternaire de formule (IIa) lié à un oxygène greffé sur le polymère de base de dextrane 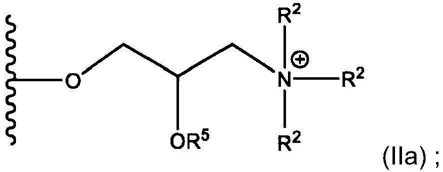 et
(ii) un groupe ammonium quaternaire de formule (IIIa) lié à un oxygène greffé sur le polymère de base de dextrane 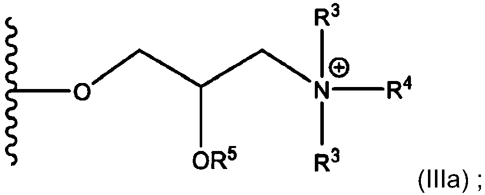
dans laquelle 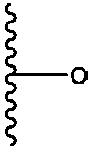 est un oxygène greffé sur le polymère de base de dextrane ;
dans laquelle chaque R² est un groupe méthyle ; dans laquelle chaque R³ est un groupe méthyle ;
dans laquelle chaque R⁴ est un groupe alkyle en C₁₂ linéaire ou ramifié ; dans laquelle chaque R⁵ est un hydrogène ; dans laquelle le polymère de conditionnement a une teneur en azote de Kjeldahl corrigée pour les cendres et les substances volatiles, TKN, >1,0 à 5,0 % en poids ; et dans laquelle le polymère de conditionnement a un degré de substitution, DS, cationique de fragments diméthyldodécyl-ammonium de 0,01 à 0,03 ; dans laquelle la formulation de conditionneur de cheveux est choisie dans le groupe constitué d'un conditionneur à laisser, d'un conditionneur à rincer et d'un shampooing conditionneur.

2. Formulation de conditionneur de cheveux selon la revendication 1, dans laquelle la formulation de conditionneur de cheveux contient moins de 0,01 % en poids, en fonction du poids de la formulation de conditionneur de cheveux, d'une huile dermatologiquement acceptable.

3. Formulation de conditionneur de cheveux selon la revendication 1, dans laquelle la formulation de conditionneur de cheveux contient moins de 0,1 % en poids, en fonction du poids de la formulation de conditionneur de cheveux, de molécules contenant du silicium.

4. Formulation de conditionneur de cheveux selon la revendication 1, comprenant en outre au moins un ingrédient additionnel choisi dans le groupe constitué d'un agent antimicrobien/conservateur ; d'un agent tensioactif nettoyant de soins capillaires ; d'un agent modifiant la rhéologie ; d'un savon ; d'un colorant ; d'un agent d'ajustement de pH ; d'un antioxydant ; d'un humectant ; d'une cire ; d'un agent moussant ; d'un agent émulsifiant ; d'un colorant ; d'un parfum ; d'un agent chélateur ; d'un conservateur ; d'un agent de blanchiment ; d'un agent lubrifiant ; d'un modificateur sensoriel ; d'un additif de protection solaire ; d'une vitamine ; d'une protéine/d'un acide aminé ; d'un extrait végétal ; d'un ingrédient naturel ; d'un agent bioactif ; d'un agent anti-vieillissement ; d'un pigment ; d'un acide ; d'un agent de pénétration ; d'un agent antistatique ; d'un agent anti-frisottis ; d'un agent antipelliculaire ; d'un agent d'ondulation/de défrisage des cheveux ; d'un agent de coiffure ; d'un absorbant ; d'une particule dure ; d'une particule molle ; d'un agent de conditionnement ; d'un agent glissant ; d'un opacifiant ; d'un agent nacrant et d'un sel.

5. Formulation de conditionneur de cheveux selon la revendication 1, comprenant en outre un agent tensioactif nettoyant de soins capillaires ; dans laquelle le polymère de conditionnement a une teneur en azote de Kjeldahl corrigée pour les cendres et les substances volatiles, TKN, de 1,4 à 2,5 % en poids ; et dans laquelle le polymère de conditionnement a un degré de substitution, DS, cationique de fragments diméthyldodécyl-ammonium de 0,01 à 0,03.

6. Procédé de conditionnement des cheveux, comprenant :
la sélection d'une formulation de conditionneur de cheveux selon la revendication 1 ; et l'application de la formulation de conditionneur de cheveux sur des cheveux.
